# EUROPEAN PATENT APPLICATION

(11) **EP 1 439 390 A2**
(43) Date of publication of application: **21.07.2004**
(21) Application number: 04250143.7
(22) Date of filing: 14.01.2004
(51) Int. Cl.: G01N 33/543, C12Q 1/68

(54) **Biosensor systems and methods of detecting biomolecular images**

(30) Priority: 15.01.2003 US 342840
(71) Applicant: Agilent Technologies, Inc., Palo Alto, CA 94306 (US)
(72) Inventor: Inaoka, Seiji, Campbell, California 995008 (US); Moon, Ronald L., Atherton, California 94027 (US)
(74) Representative: Tollett, Ian

(57) **Abstract**

Methods and systems for amplifying biomolecule images are disclosed. Briefly described, a representative system provides for a biosensor system (100) for amplifying an image of a first biomolecule (110). The system (100) includes a nanoparticle (114) disposed on the first biomolecule (110) and a polymer (124) disposed on the nanoparticle (114). The polymer (124) is catalytically generated on the nanoparticle (114) from a plurality of monomers (118). In addition, the system (100) includes a detector for detecting the first biomolecule (110).

## Description

The present invention relates to a method of detecting biomolecules and a biosensor system for amplifying an image of a biomolecule.

In general, a biosensor is capable of identifying biomolecules such as polynucleotides and polypeptides. One use of a biosensor has been to deposit probes onto a substrate and place a biomolecule sample containing target biomolecules onto the substrate. The probe can be various biomolecules that can interact with target biomolecules, while the substrate can be a solid surface such as silica, surface-derivatized glass, polypropylene, and activated polyacrylamide. Then, the target biomolecules can interact (*e.g.*, bond or hybridize) with one or more of the probes. Subsequently, the interaction can be analyzed based on the presence and location of the target biomolecule-probe interaction, which can be determined by one or more detection techniques such as optical, radiochemical, and electrochemical techniques.

Another way in which a biosensor is used includes depositing target biomolecules onto a substrate and adding a solution. The solution can include complimentary polynucleotide or polypeptide probe samples derived from biomolecules that have been tagged with fluorescent dyes. The probe material interacts selectively with target biomolecues only where complimentary bonding sites occur. In other words, probe biomolecules with similar chemical characteristics (*e.g.*, nucleotide sequence or amino acid sequence) to the target biomolecule interact with the target molecules, while dissimilar probe biomolecules do not significantly interact with the target biomolecules. Thereafter, the presence and quantity of bound probe biomolecules can be detected and analyzed in a manner similar to the detection techniques discussed above.

As stated above, biomolecules can be detected using a variety of detection techniques. However, many of these techniques have disadvantages such as low detection limits, variation in reproducibility of results, and problems with specificity of intercalations. Thus, there is a need in the industry for a biomolecule detection technique that overcomes at least these disadvantages.

Embodiments of the present invention provide methods and systems for detecting biomolecular images. Briefly described, one embodiment of the system, among others, provides a biosensor system for amplifying an image of a first biomolecule. The system includes a nanoparticle disposed on the first biomolecule and a polymer disposed on the nanoparticle. The polymer is catalytically generated on the nanoparticle from a plurality of monomers. In addition, the system includes a detector for detecting the polymer.

Embodiments of the present invention also include methods for detecting a biomolecular image. In this regard, one embodiment of such a method, among others, can be broadly summarized by the following steps: providing a first biomolecule, providing a first nanoparticle, forming a target complex that includes the first nanoparticle and the first biomolecule, forming a biomolecular image that includes the target complex; and amplifying the biomolecular image by catalytically forming a polymer on the first nanoparticle of the target complex.

Aspects of a number of preferred embodiments of the present invention can be better understood with reference to the following drawings. The components in the drawings are not necessarily to scale, emphasis instead being placed upon clearly illustrating a number of preferred embodiments of the present invention. Moreover, in the drawings, like reference numerals designate corresponding parts throughout the several views.
FIG. 1 is a flow diagram illustrating a representative embodiment of the amplification process of the present invention.
FIG. 2 is a flow diagram illustrating a representative embodiment of the chemical amplification process of the present invention.
FIG. 3 is a flow diagram illustrating a representative flow of an aspect of the chemical amplification process shown in FIG. 1.
FIGS. 4A through 4E illustrate a representative schematic diagram of a process for chemically amplifying a biomolecular image.
FIGS. 5A through 5E illustrate another representative schematic diagram of a process for chemically amplifying a biomolecular image.
FIG. 6 is a graph illustrating an embodiment of chemical amplification as a function of an amount of monomer used during the amplification process.
FIG. 7 is a graph illustrating an embodiment of chemical amplification as a function of an amount of oxidant used during the amplification process.
FIG. 8 is a graph illustrating an embodiment of chemical amplification as a function of pH.
FIG. 9 is a flow diagram illustrating a representative embodiment of the electrochemical amplification process of the present invention.
FIG. 10 is a flow diagram illustrating a representative flow of an aspect of the electrochemical amplification process shown in FIG. 1.
FIGS. 11A through 11D illustrate a representative schematic diagram of a process for electrochemically amplifying a biomolecular image.

Embodiments of the present invention provide biosensors capable of detecting, for example, biomolecules such as deoxyribouncleic acid (DNA), ribonucleic acid (RNA), nucleotides, oligonucleotides, nucleosides, proteins, peptides, and polypeptides. In particular, embodiments of the present invention provide for systems and methods for amplifying *(i.e.,* chemically and electrochemically) an image of a biomolecule to enhance the detection capabilities of the biosensor. The image can be described as an image of a molecule or a group of molecules on a substrate like a silica substrate.

In general, amplification of the image of the biomolecule (biomolecular image) includes selectively causing a biological interaction to occur between a nanoparticle and the biomolecule of interest forming a nanoparticle/biomolecule complex. Amplification can be defined as causing the image of the biomolecule to appear larger and more optically visible than it would alone. A biological interaction can be defined as bonding or hybridization between two biological functional groups (*e.g.*, biotin-avidin (streptavidin) interaction). The nanoparticle of the nanoparticle/biomolecule complex is used as a nucleation site to catalytically form a polymer on the nanoparticle. The polymer has a high optical density, which thereby enhances the ability to detect the biomolecule by detection of the polymer. In other words, the detected image of the polymer disposed on the nanoparticle/biomolecule complex corresponds to the biomolecular image. Thus, the biomolecular image is amplified because the polymer has a higher optical density than the biomolecule alone. Therefore, the biomolecule can be qualitatively identified and/or quantitatively measured by detection of the polymer.

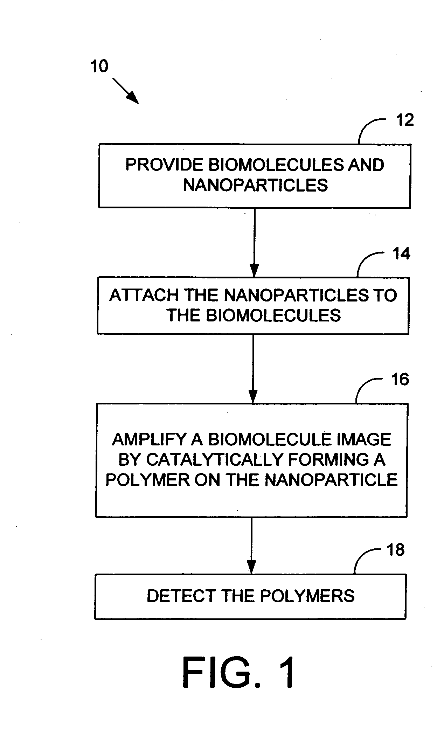

FIG. 1 is a flow diagram illustrating an exemplary process 10 for amplifying a biomolecular image. In block 12, biomolecules and nanoparticles are provided in a vessel. The biomolecules can be located in solution or attached to a substrate. Additional details regarding the biomolecules and nanoparticles will be discussed below. In block 14, the nanoparticles are attached (*e.g.*, biologically bonded or hybridized) to the biomolecules. In block 16, the biomolecular image is amplified by forming a polymer on the nanoparticle. In block 18, after forming the polymer on the nanoparticle, the polymer can be detected using a detection system. Detection systems can include, for example, optical detection techniques (*e.g.* fluorescence), radiochemical detection techniques (*e.g.*, radiochemical tags), and electrochemical detection techniques (*e.g.*, detection of electroactive species).

The amplification of the biomolecular image can be performed through chemical or electrochemical processes. The remainder of this disclosure will first describe chemical amplification of the biomolecular image and then describe electrochemical amplification of the biomolecular image.

### Chemical Amplification

In general, chemical amplification of a biomolecular image includes selectively causing a biological interaction (*e.g.*, bonding or hybridization) to occur between a nanoparticle and the biomolecule of interest forming a nanoparticle/biomolecule complex. The nanoparticle is used as a nucleation site to catalytically *(i.e.,* chemically via an oxidant) form a polymer on the nanoparticle. The polymer has a high optical density, which thereby enhances the ability to detect the biomolecule by detection of the polymer. In other words, the detected image of the polymer disposed on the nanoparticle/biomolecule complex corresponds to the bimolecular image. Therefore, the biomolecule can be qualitatively identified and/or quantitatively measured by detection of the polymer.

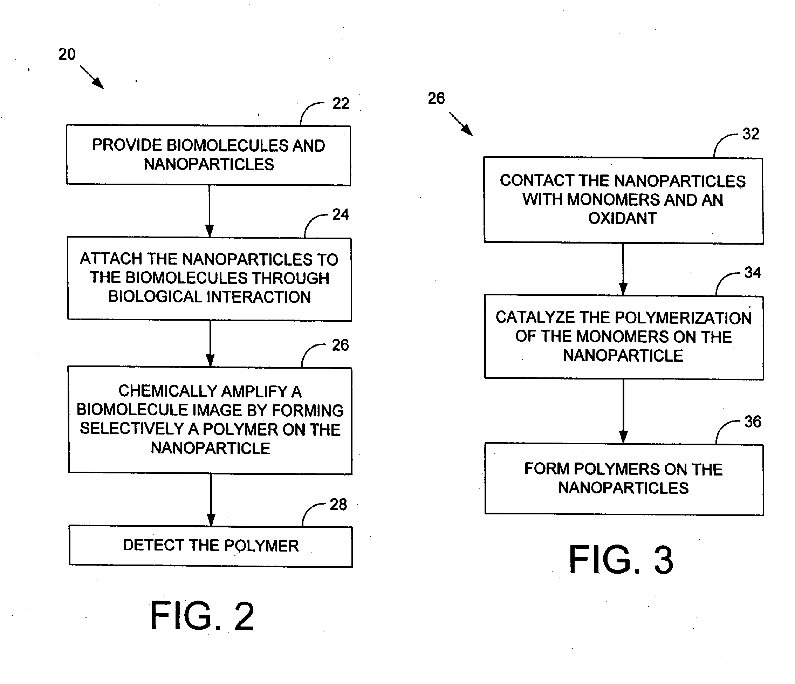

FIG. 2 is a flow diagram illustrating an exemplary process 20 for chemically amplifying a biomolecular image. In block 22, biomolecules and nanoparticles are provided in a vessel. The biomolecules can be located in solution or attached to a substrate. Additional details regarding the biomolecules and nanoparticles will be discussed below. In block 24, the nanoparticles are disposed (*e.g.*, biologically bonded or hybridized) on the biomolecules through a biological interaction. In block 26, the biomolecular image is chemically amplified by selectively forming a polymer on the nanoparticle. In block 28, after forming the polymer on the nanoparticle, the polymer can be detected using a detection system such as, but not limited to, optical, radiochemical, and electrochemical techniques.

FIG. 3 is a flow diagram illustrating an exemplary aspect of the flow diagram shown in FIG. 2. In particular, FIG. 3 is a flow diagram illustrating the steps of chemically amplifying the biomolecular image, as shown in block 26. In this regard, a solution having monomers and an oxidant is brought into contact with the nanoparticles disposed (*e.g.*, biologically bonded or hybridized) on the biomolecules, as shown in block 32. The solution of monomers and the oxidant can contact the biomolecules sequentially or simultaneously. Additional details regarding the monomers and oxidants are discussed below. In block 34, the oxidant acts as a catalyst and initiates the polymerization of the monomers on the surface of the nanoparticle. As a result, polymers, which can then be detected, are formed on the nanoparticles, as shown in block 36.

Having described biosensors and chemical amplification of biomolecular images in general, five examples follow that describe some embodiments of the systems and methods of using a biosensor to chemically amplify a biomolecular image. While embodiments of the biosensor and the chemical amplification of the biomolecular image are described in connection with the following examples and the corresponding text, there is no intent to limit embodiments of the biosensor and the chemical amplification of the biomolecular image to these examples. On the contrary, the intent is to cover all alternatives, modifications, and equivalents included within the scope of the invention.

### Example 1

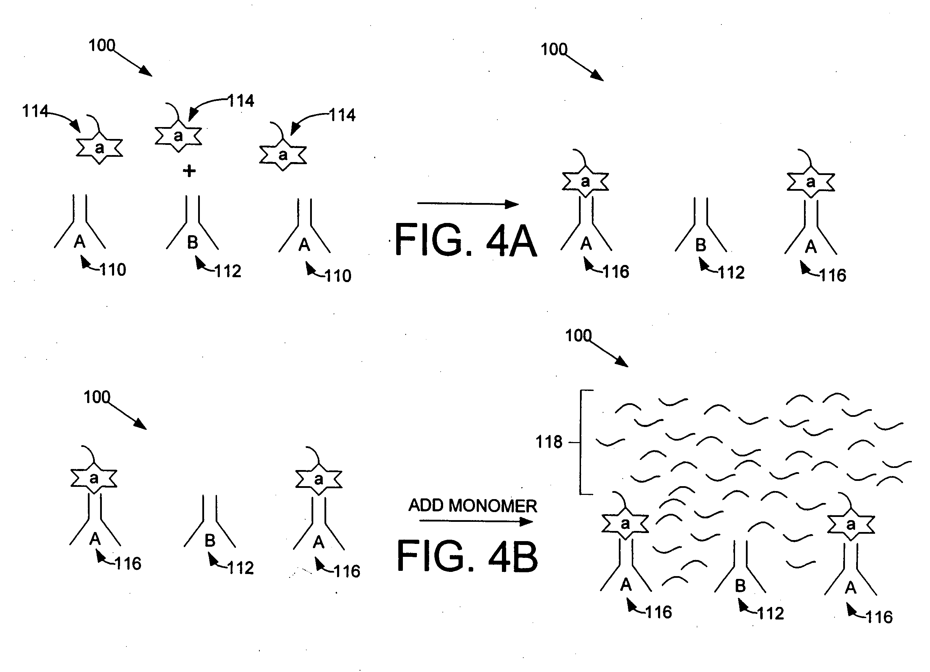

FIGS. 4A through 4E are exemplary schematic diagrams of a process for chemically amplifying a target biomolecular image for a biosensor 100. FIG. 4A illustrates biosensor 100, where target biomolecules (A) 110 and non-target biomolecule (B) 112 are brought into contact with nanoparticles 114. As indicated above, target biomolecules (A) 110 can include, for example, DNA, RNA, nucleotides, oligonucleotides, nucleosides, proteins, peptides, and polypeptides. The nanoparticles 114 have an affinity for target biomolecule (A) 110 and, therefore, are preferentially attracted to the target biomolecule (A) 110 rather than non-target biomolecule B 112. Consequently, the nanoparticles 114 are disposed on target biomolecules (A) 110, thereby forming target complex 116 *(i.e.,* the unamplified biomolecular image).

The nanoparticles 114 can be disposed on target biomolecules (A) 110 by selectively interacting (*e.g.*, biological bonding or hybridization) with target biomolecules (A) 110. The nanoparticles 114 can have a moiety and/or one or more functional groups disposed on the nanoparticle 114 that have an affinity for a particular biomolecule type(s). Therefore, biosensors can be designed to chemically amplify one or more biomolecules by exposing the biomolecules to one or more nanoparticles having a known affinity for the particular biomolecule of interest. In particular, the nanoparticles 114 can be conjugated polymer nanoparticles such as, for example, 3,4-dioxyethylenethiophene (EDOT)/3-(N-pyrroyl) propionic acid in the size range of about 1 nanometer to about 1000 nanometers.

FIG. 4B is a schematic diagram illustrating a solution containing monomers 118 coming into contact with target complexes 116 and non-target biomolecule 112. The monomers 118 can be any monomer that can be formed into a polymer on the nanoparticle 114 disposed on the target complex 116. The monomers 118 in solution can include one or more monomers 118 such as, for example, a pyrroyl complex, a thiophene complex, an aniline complex, a fluorene complex, a carbazole complex, and a catechol (1,2-benzenediol) complex.

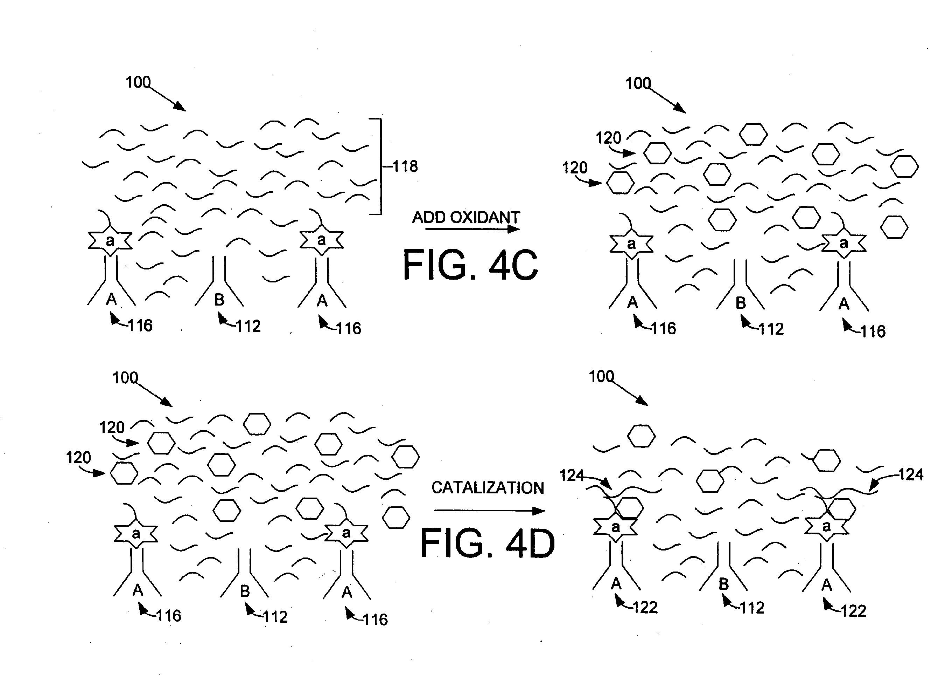

FIG. 4C is a schematic diagram illustrating the interaction of an oxidant 120 to the monomers 118. The oxidant comes into contact with target complexes 116, and non-target biomolecule 112. The oxidant 120 can include, for example, transition metal salts and persulfate compounds. In particular, the oxidant 120 can include iron (III) chloride, ammonium persulfate, iron acetylacetonate, and potassium ferricyanide. The oxidant 120 is added to the solution containing monomers 118, target complexes 116, and non-target biomolecules 112 to initiate the polymerization of the monomers 118 into polymers (polymer 124, shown in FIG. 4D) on the nanoparticle disposed on the target complexes 116. In this manner, the nanoparticle acts as a nucleation site for the formation of the polymer.

FIG. 4D is a schematic diagram illustrating the polymerization of the monomers 118 into polymers 124, thereby forming target polymer complex 122 *(i.e.,* the amplified biomolecular image). In particular, the polymerization process is catalyzed by the oxidant 120 interacting with the nanoparticles 114 located on the target polymer complex 122. The polymers 124 formed on the target polymer complex 122 can be any polymer formed by the polymerization of the monomers or combination of monomers. In particular, the polymer 124 can include, but is not limited to, polypyrroyl, polythiophene, polyaniline, polyfluorene, polycarbazole, polycatechol (1,2-benzenediol), and combinations thereof.

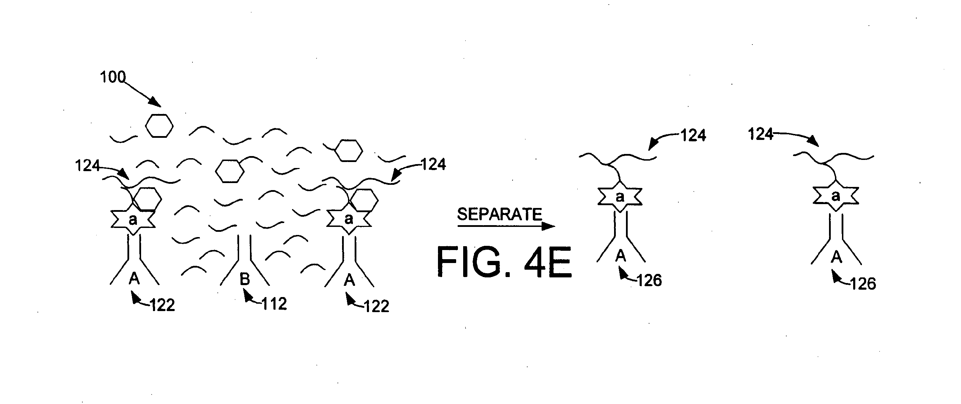

FIG. 4E is a schematic diagram illustrating the separation (*e.g.*, rinsing) of the monomer 118, oxidant 120, non-target biomolecule 112, and the remaining solution from target polymer complex 122. Additional preparation and/or treatment processes of target polymer complex 122 can be performed to facilitate the detection of the target polymer complex 122. For example, target polymer complex 122 can be disposed upon a substrate to facilitate detection. In addition, target polymer complex 122 can be exposed to a chemical and/or an irradiation process that enhances the ability to detect the polymer. The target polymer complex 122 can then be detected using detection systems capable of detecting the polymers 124 on target polymer complex 122, the nanoparticles 114, and/or target biomolecules 110.

The detection system can use detection techniques that include, for example, optical, radiochemical, and electrochemical detection techniques. In some instances, the polymer 124 on the target polymer complex 122 can be viewed with the human eye, thereby facilitating an inexpensive qualitative detection technique. For example, when a biosensor is designed to form polymers only on a specific type of target biomolecule, visual detection of the polymer confirms the presence of the target biomolecule. Such a detection technique may be useful during experimentation where other detection techniques are impractical.

The concentration of the oxidant 120 and the monomer 118 depend upon, at least, the chemical formulation and concentration of the oxidant 120, the chemical formulation of the monomer 118, the concentration of the monomer 118, the pH of the solution, the concentration of acid to achieve the appropriate pH, the temperature of the solution, the type of nanoparticle 114, and the concentration of the target biomolecule. Therefore, the conditions present while performing chemical amplification of the target biomolecular image can vary depending upon the design of the biosensor 100. Examples 2 through 5 below illustrate exemplary conditions for designing bisensors to achieve appropriate biomolecular image chemical amplification.

### Example 2

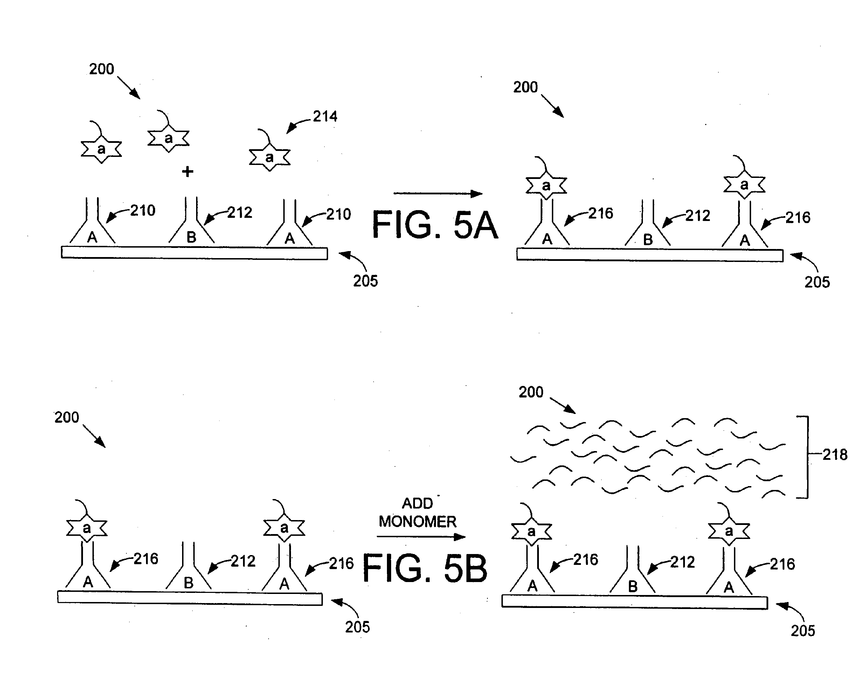

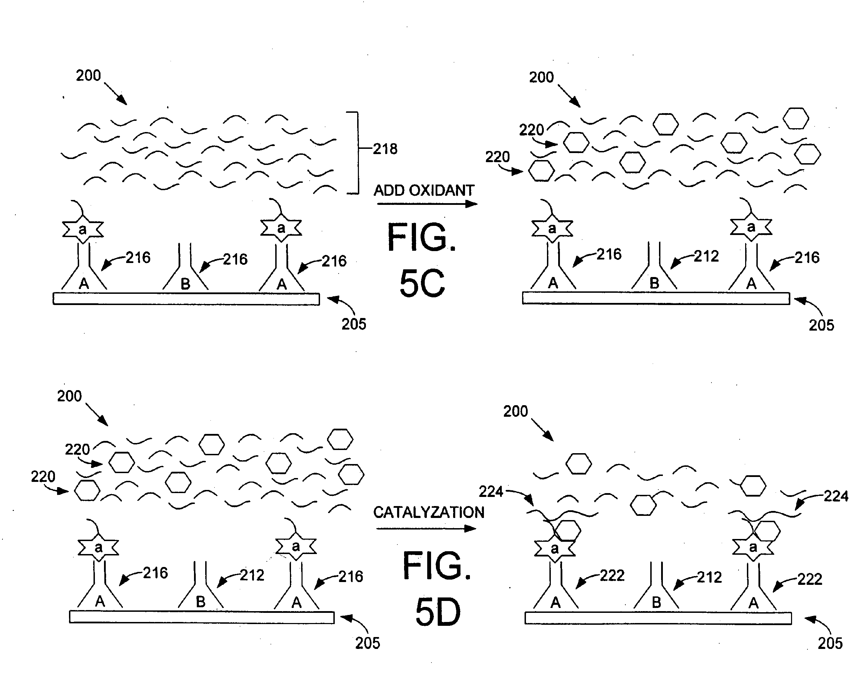

FIGS. 5A through 5E are exemplary schematic diagrams collectively illustrating a process for amplifying a target biomolecular image for a biosensor 200. FIG. 5A illustrates biosensor 200, where target biomolecules (A) 210 and non-target biomolecule (B) 212 are disposed onto a substrate 205. In addition, FIG. 5A illustrates the introduction of nanoparticle 214 to target biomolecules (A) 210 and non-target biomolecule (B) 212. Target biomolecules (A) 210 are similar to target biomolecules (A) 110 discussed in reference to FIGS. 4A through 4E.

The substrate 205 can be any substrate that has an affinity for target biomolecules (A) 210 and non-target biomolecule (B) 212. For example the substrate maybe silica, surface-derivatized glass, polypropylene, and activated polyacrylanide. In particular, the substrate 205 can include glass slides modified with aminopropylsilane. Other substrates can be designed to have an affinity for one or more biomolecules. Therefore, suitable substrates for biosensors can be designed to interact only with one biomolecule. In addition, the surface of the substrate can be designed to interact with specific biomolecules at one or more predetermined positions on the substrate.

The nanoparticles 214 are similar to the nanoparticles 114 discussed in reference to FIGS. 4A through 4E. In this regard, the nanoparticles 214 have a preferential affinity for target biomolecule (A) 210 rather than non-target biomolecule (B) 212. Consequently, the nanoparticles 214 are immobilized on target biomolecules (A) 210, thereby forming target complex 216 *(i.e.,* the unamplified biomolecular image).

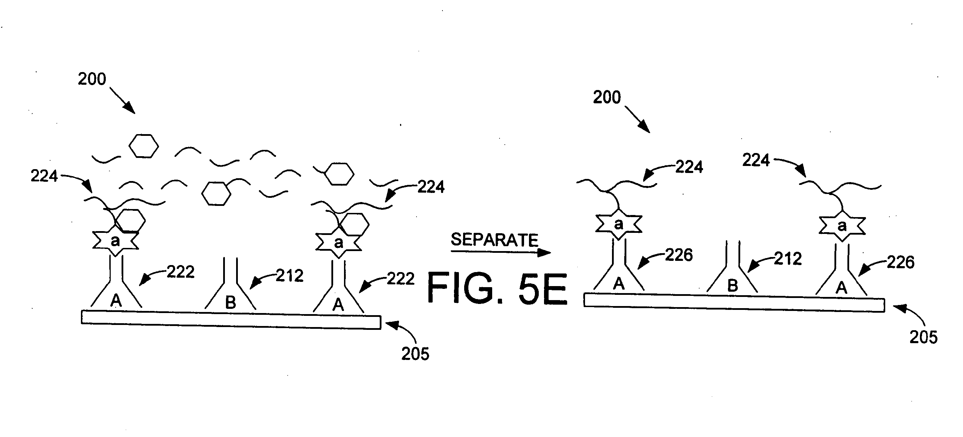

FIG. 5B is a schematic diagram illustrating the introduction of a solution containing monomers 218 to contact with target complexes 216 and non-target biomolecule 212. The monomers 218 are similar to the monomers 118 discussed in reference to FIGS. 4A-4E.

FIG. 5C is a schematic diagram illustrating the introduction of the oxidant 220 to the monomers 218, and target complexes 216 and non-target biomolecule 212, disposed on the substrate 205. The oxidant 220 is similar to the oxidant 120 referred to in FIGS. 4A through 4E. The oxidant 220 is added to the solution containing monomers 218 and target complexes 216 and non-target biomolecules 212 disposed on the substrate 205 to initiate the polymerization of the monomers 218 into polymers (polymer 224, shown in FIG. 5D) on the nanoparticles 214.

FIG. 5D is a schematic diagram illustrating the polymerization of the monomers 218 into polymers 224, thereby forming target polymer complex 222 on the substrate 205 *(i.e.,* the amplified biomolecular image). In particular, the polymerization process is catalyzed by the oxidant 220 interacting with the nanoparticles 214, which act as nucleation sites, located on the target polymer complex 222. The polymer 224 is formed on the target polymer complex 222 is similar to the polymer 124 referred to in FIGS. 4A through 4E.

FIG. 5E is a schematic diagram illustrating the separation (*e.g.*, rinsing) of the monomer 218, oxidant 220, and the remaining solution from the target polymer complexes 222 and non-target biomolecules 212 disposed on the substrate 205. As discussed above, additional preparation and/or treatment of the target polymer complexes 222 can be performed to facilitate the detection of the target polymer complexes 222.

The target polymer complexes 222 can be detected using one or more detection systems capable of detecting the polymers 224 on the target polymer complexes 222, the nanoparticles 214, and/or the target biomolecules 210. The detection system can include detection techniques discussed above in reference to FIGS. 4A through 4E.

As discussed in reference to FIGS. 4A through 4E, the concentrations of the oxidant 220 and the monomer 218 depend upon, at least, the chemical formulation of the oxidant 218, the concentration of the oxidant 220, the chemical formulation of the monomer 218, the concentration of the monomer 218, the pH of the solution, the concentration of acid to achieve the appropriate pH, the temperature of the solution, the type of nanoparticle 214, the substrate 205, and the concentration of the target biomolecule. Therefore, the conditions present while amplifying of the target biomolecular image can vary depending upon the design of the biosensor 200.

### Example 3

In examples 3, 4, and 5, the substrate having nanoparticles disposed thereon (as a substantially circular spot) is placed in a vessel having a solution including monomers and an acid. The substrate is a aminopropylsilane modified glass slide, while the nanoparticles are silica conjugated polymer nanoparticles. The monomer is 3-N-pyrrolyl propionic acid (18 M), while the acid is hydrochloric acid (12 M). The oxidant *(i.e.,* 100 milligrams of iron chloride (6.2 M)) is added to the vessel to initiate polymerization of the monomers into polymers on the nanoparticles. The amount of monomer, acid, and oxidant is varied for examples 3, 4, and 5, respectively.

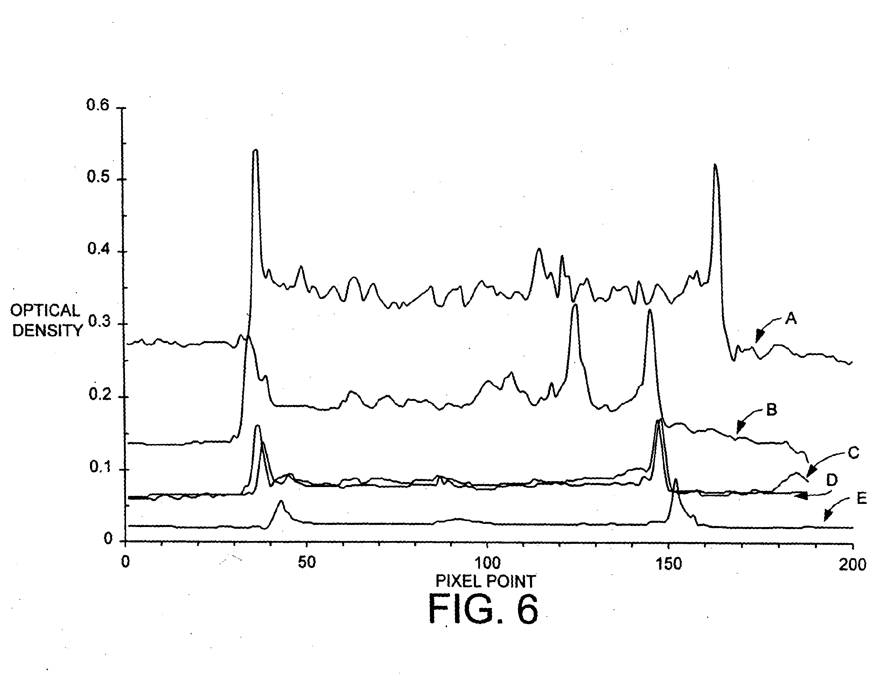

After sufficient time, which can be approximately three hours, the substrate is removed from the vessel and analyzed to determine the degree of polymerization on the nanoparticle. An image of the substrate having polymer disposed on the nanoparticles can be obtained using an imaging device such as, but not limited to, a standard flatbed scanner including Leaf EasyScan^{TM}, or any digital camera, either of which can be controlled using a standard personal computer. Subsequently, the scanned image can be analyzed using ImagePro^{TM} software, which can determine the densitometry of the scanned image data. The densitometry data is a profile, of pixel points, of the area of the image (the spot) as a function of optical density. A pixel point can be defined as picture elements that define a unit area of the image. In other words, each pixel point has a corresponding optical density. Thus, the graphs in FIGS. 6-8 illustrate the optical density of the image for each pixel point.

FIGS. 6 through 8 measure the change in the optical density of the polymer at each pixel point as a function of the concentration of a monomer, an oxidant, and an acid. FIGS. 6-8 includes graphs having four or five curves, where the vertical axis illustrates optical density and the horizontal axis illustrates pixel points. The curves illustrate the amount of polymer that is formed on an area of the substrate having nanoparticles disposed thereon. The polymer is measured by optical density and the area is a portion of the spot as measure by pixel points. Thus, the greater the amount of polymer formed on the nanoparticle, the higher the position of the curve on the vertical axis.

As will be observed in the various data sets on each figure, the size and shape of the curves are different. The respective differences in the curves can be attributed to hand-spotting the solution onto the substrate, which causes the spots to have different sizes, and the density of the polymers at different parts of the spot. However, the increase in optical density illustrates the amplification of the image.

FIG. 6 is a graph illustrating chemical amplification as a function of the monomer added in the solution. The vertical axis of the graph illustrates the optical density of the polymer, which is a measure of the ability of the polymer to absorb electromagnetic energy incident upon the polymer. The horizontal axis illustrates density data at particular pixel points.

In this example, the monomer is a pyrroyl and the nanoparticle is a silica polymer (EDOT/3(N-pyrroyl) propionic acid) conjugated nanoparticle. The amount of the pyrroyl used in the experiments ranges from 0 to about 20 microliters, while the amount of oxidant *(i.e.,* 100 milligrams of iron (III) chloride (FeCl₃)) and acid *(i.e.,* 15 microliters of 12-M hydrochloric acid (HCl)) is held constant. In this regard, curves "A," "B," "C," "D," and "E" measure the optical density of the polymer formed on the nanoparticle for the following amounts of the pyrroyl, respectively: 20 microliters of 18 M pyrroyl; 10 microliters of 18 M pyrroyl; 5 microliters of 18 M pyrroy; 2 microliters of 18 M pyrroyl; and 0 microliters the pyrroyl. As shown by the graph, the chemical amplification of the polymer increases as the amount of the pyrroyl added increases *(i.e.,* the curve is positioned at higher values on the vertical axis). Although curve "A" represents the greatest increase in optical density, curves "B," "C," and "D," and "E" represent conditions that are suitable for biosensors.

### Example 4

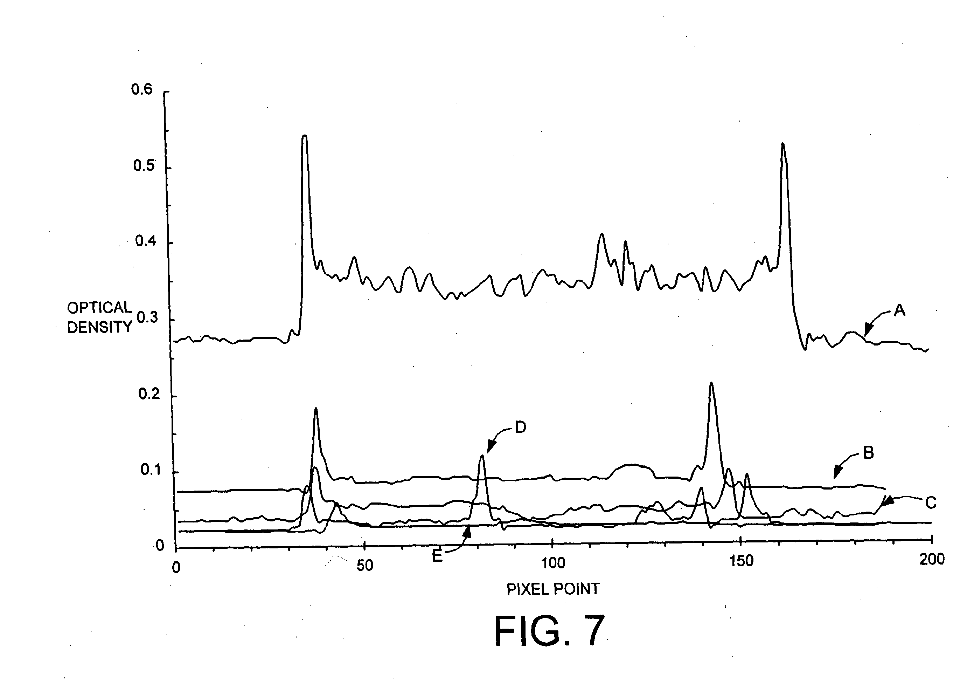

FIG. 7 is a graph illustrating chemical amplification as a function of the oxidant added in the solution. The vertical axis of the graph illustrates optical density, while the horizontal axis illustrates pixel points. In this example, the oxidant is FeCl₃ and the nanoparticle is a silica polymer (EDOT/3(N-pyrroyl) propionic acid) conjugated nanoparticle. The amount of FeCl₃ used in the experiments ranges from 0 to about 100 milligrams, while the amount of monomer (*i.e.*, 20 microliters of 18 M pyrroyl) and acid *(i.e.,* 15 microliters of 12 M HCl) are held constant. In this regard, curves "A," "B," "C," "D," and "E" measure the optical density of the polymer formed on the nanoparticle for the following amounts of FeCl₃, respectively: 100 milligrams of FeCl₃ (0.62 millimoles); 33.3 milligrams of FeCl₃ (0.20 millimoles); 13.3 milligrams FeCl₃ (0.082 millimoles); 6.67 milligrams of FeCl₃ (0.041 millimoles); and 0 milligrams of FeCl₃.

As shown by the graph, the chemical amplification of the polymer increases as the amount of FeCl₃ added increases (*i.e.*, the curve is positioned at higher values on the vertical axis). Although curve "A" represents the greatest increase in optical density, curves "B," "C," "D," and "E" represent conditions that are suitable for biosensors.

### Example 5

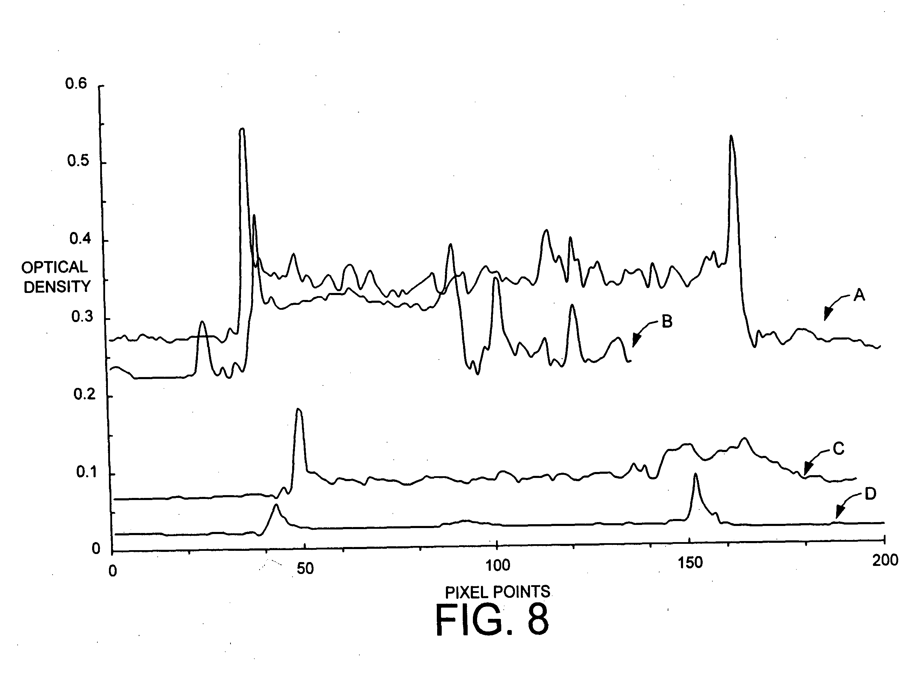

FIG. 8 is a graph illustrating chemical amplification as a function of the acidity *(i. e.,* pH) of the solution. The vertical axis of the graph illustrates the optical density, while the horizontal axis illustrates pixel points. In this example, the acid for adjusting the pH is HCl and the nanoparticle is a silica polymer (EDOT/3(N-pyrroyl) propionic acid) conjugated nanoparticle. The amount of HCl used in the experiments ranges from 0 to about 1500 microliters, while the amount of monomer (*i.e.*, 20 microliters of 18 M pyrroyl) and oxidant *(i.e.,* 100 milligrams of FeCl₃) are held constant. In this regard, curves "A," "B," "C," and "D" measure the optical density of the polymer formed on the nanoparticle for the following amounts of HCl, respectively: 15 microliters of 12 M HCl (about a pH of 2.00); 150 microliters of 12 M HCl (about a pH of 1.42); 1500 microliters of 12 M HCl (about a pH of 0.61); and 0 microliters of 12 M HCl (about a pH of 2.12).

As shown by the graph, the chemical amplification of the polymer increases as the amount of HCl added decreases but greater than zero *(i.e.,* the curve is positioned at higher values on the vertical axis). Although curve "A" represents the greatest increase in optical density, curves "B," "C," and "D" represent conditions that are suitable for biosensors.

The results obtained in examples 3-5 can be used to design biosensors. For example, curve "A" in FIGS. 6, 7, and 8 represents the greatest increase in optical density for the particular quantities of monomer, oxidant, and acid, respectively. Therefore, a biosensor can be designed to chemically amplify biomolecular images, where the monomer, oxidant, and acid are as follows: about 20 microliters of 18 M pyrroyl, about 15 microliters of 12 M HCl, and about 100 milligrams of 6.2 M FeCl₃, respectively. However, other conditions may be appropriate for particular applications.

### Electrochemical Amplification

In general, electrochemical amplification of a biomolecular image includes selectively causing a biological interaction (*e.g.*, bonding or hybridization) to occur between a nanoparticle and the biomolecule of interest, thus forming a nanoparticle/biomolecule complex. The nanoparticle is used as a nucleation site to catalytically *(i.e.,* electrochemically) form a polymer on the nanoparticle. The polymer has a high optical density, which thereby enhances the ability to detect the biomolecule by detection of the polymer. In other words, the detected image of the polymer disposed on the nanoparticle/biomolecule complex corresponds to the biomolecular image. Therefore, the biomolecule can be qualitatively identified and/or quantitatively measured by detection of the polymer.

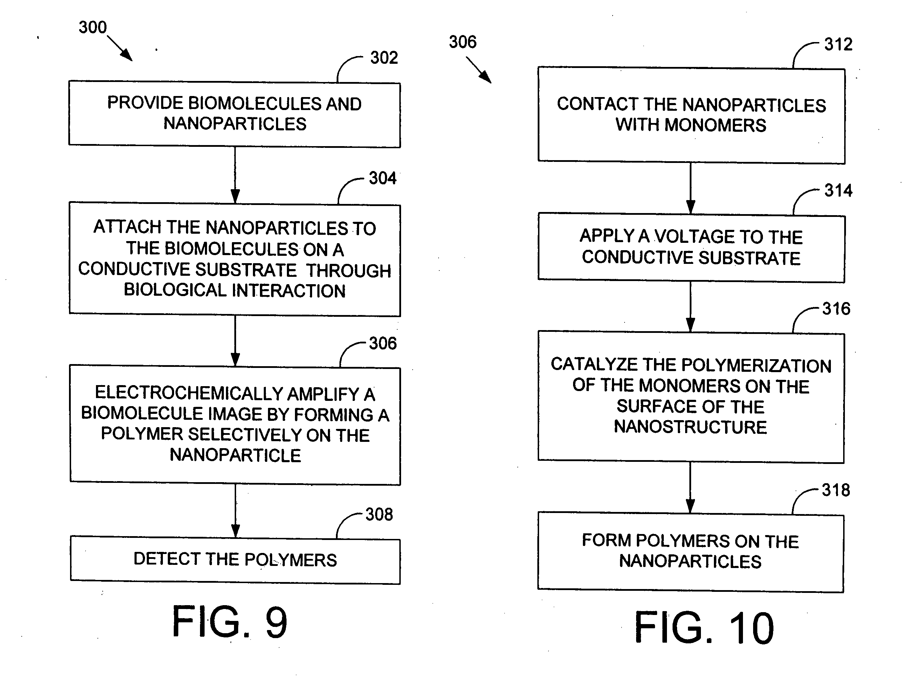

FIG. 9 is a flow diagram illustrating an exemplary process 300 for electrochemically amplifying a biomolecular image. In block 302, biomolecules and nanoparticles are provided in a vessel. The biomolecules are attached to a conductive support such as, for example, gold, indium tin oxide (ITO), or a different material. Additional details regarding the biomolecules, nanoparticles, and conductive supports will be discussed below. In block 304, the nanoparticles are disposed (*e.g.*, biologically bonded or hybridized) on the biomolecules through a biological interaction. In block 306, the biomolecular image is electrochemically amplified by selectively forming a polymer on the nanoparticle. In block 308, after forming the polymer on the nanoparticle, the polymer is detected using a detection system such as optical, radiochemical, and electrochemical techniques.

FIG. 10 is a flow diagram illustrating an exemplary aspect of the flow diagram shown in FIG. 9. In particular, FIG. 10 illustrates a flow diagram illustrating the steps of electrochemically amplifying the biomolecular image, as shown in block 306. In this regard, a solution having monomers is brought into contact with the nanoparticles disposed (*e.g.*, biologically bonded or hybridized) on the biomolecules, as shown in block 312. Additional details regarding the monomers are discussed below. In block 314, a voltage is applied to the conductive support. In block 316, the voltage catalyzes the polymerization of the monomers on the nanoparticle. As a result, polymers, which can then be detected, are formed on the nanoparticles, as shown in block 318.

Having described biosensors and electrochemical amplification of biomolecular images in general, an example follows that describes an embodiment of a system and a method of using a biosensor to electrochemically amplify biomolecular images. While an embodiment of the biosensor and the electrochemical amplification of the biomolecular image are described in connection with the following example and the corresponding text, there is no intent to limit the embodiment of the biosensor and the electrochemical amplification of the biomolecular image to this example. On the contrary, the intent is to cover all alternatives, modifications, and equivalents included within the scope of the invention.

### Example 6

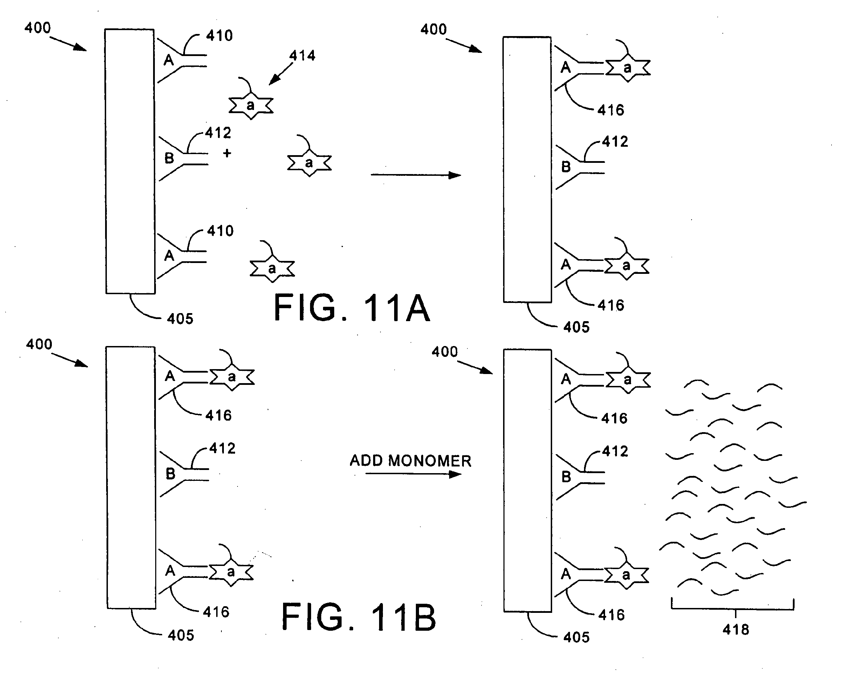

FIGS. 11A through 11D are exemplary schematic diagrams collectively illustrating a process for electrochemically amplifying a target biomolecular image for a biosensor 400. FIG. 11A illustrates biosensor 400, where target biomolecules (A) 410 and non-target biomolecule (B) 412 are disposed onto a conductive support 405. In addition, FIG. 11A illustrates the introduction of nanoparticle 414 to target biomolecules (A) 410 and non-target biomolecule (B) 412. Target biomolecules (A) 410 are similar to target biomolecules (A) 110 discussed in reference to FIGS. 4A through 4E. The concentrations of the reagents correspond to those discussed in Examples 1-5.

The conductive support 405 can be any conductive support that has an affinity for target biomolecules (A) 410 and non-target biomolecule (B) 412. For example the conductive support 405 may be formed of, for example, gold or indium tin oxide (ITO). In addition, the conductive support 405 can be designed to have an affinity for one or more biomolecules. Therefore, biosensors can include conductive supports that interact only with one biomolecule. Furthermore, the surface of the conductive support can be designed to interact with specific biomolecules at predetermined positions on the conductive support.

The nanoparticles 414 are similar to the nanoparticles 114 discussed in reference to FIGS. 4A through 4E. In this regard, the nanoparticles 414 have a preferential affinity for target biomolecules (A) 410 rather than non-target biomolecule (B) 412. Consequently, the nanoparticles 414 are immobilized on target biomolecules (A) 410, thereby forming target complexes 416 *(i.e.,* the unamplified biomolecular image).

FIG. 11B is a schematic diagram illustrating the introduction of a solution containing monomers 418 to target complexes 416 and non-target biomolecule 412. The monomers 418 are similar to the monomers 118 discussed in reference to FIGS. 4A through 4E.

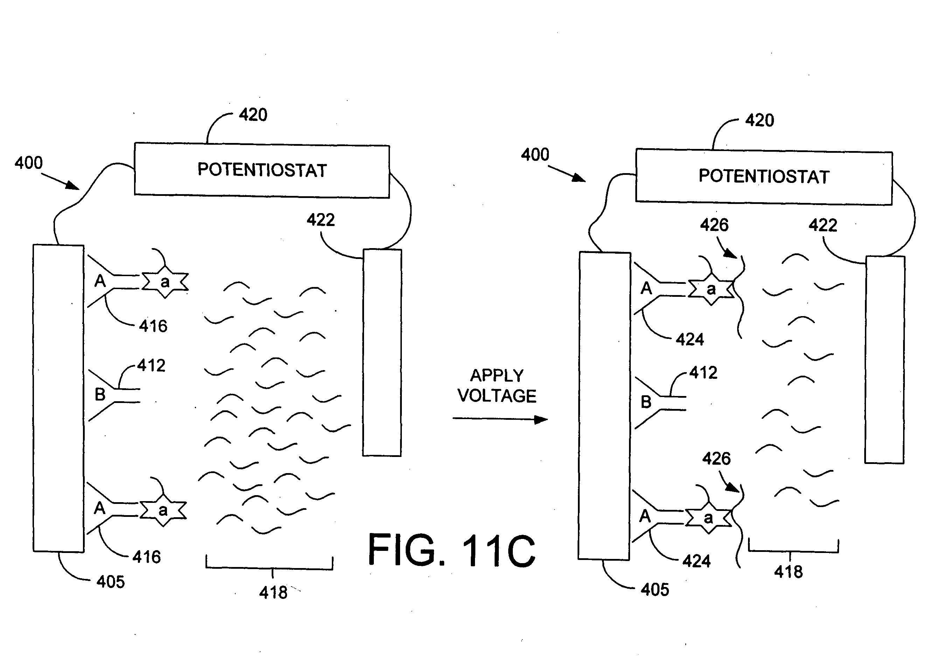

FIG. 11C is a schematic diagram illustrating applying a voltage to the conductive support 405 in the presence of the monomers 418 and target complexes 416 and non-target biomolecule 412. The voltage (anodic) can be applied by a potentiostat 420 having a counter electrode 422 and a reference electrode (not shown). The reference electrode can include silver/silver chloride (Ag/AgCl). The applied voltage initiates the polymerization of the monomers 418 into polymers 426 on the target complexes 416, thereby forming target polymer complexes 424 on the substrate 405. (General reference, Bard, *et al., Electrochemical Methods: Fundamentals and Application,* 2^{nd} Ed. 2000.)

In particular, the nanoparticle 414 is oxidized when the anodic voltage is applied to the conductive support 405. The nanoparticles 414 are made so that they have a lower oxidation potential than monomers 418. Therefore, the nanoparticles 414 are oxidized at a lower anodic voltage potential than the monomers 418. This phenomenon is utilized to make the nanoparticles 414 act as nucleation sites for the polymerization, and as a result, the polymer 426 selectively forms at the location where nanoparticles 414 are immobilized.

The polymer 426 formed on the target polymer complex 424 is similar to the polymer 124 referred to in FIGS. 4A through 4E. Using common conjugated polymers such as, but not limited to, can preserve the original distribution (image) of nanoparticles 414 on the surface of the conductive support 405. In addition, the common conjugated polymers have a high optical density, which can be used to enhance (amplify) the intensity of the original image by forming polymers 426 on the nanoparticles 414.

The potentiostat 420 can control the polymerization by adjusting the applied voltage, the scanning speed of the cyclic voltammogram, and the number of scanning cycles. In general, the applied voltage can range between about 100 and 1500 millivolts (versus Ag/AgCl reference electrode), between about 400 and 1300 millivolts (versus Ag/AgCl), and between about 900 and about 1200 millivolts (versus Ag/AgCl). The voltammogram scan rate can vary from about 10 millivolts per second to about 300 millivolts per second and from about 50 millivolts per second to about 200 millivolts per second. In particular, the applied voltage can be about 1200 millivolts and the scan rate can be about 50 millivolts per second. It should be noted that the applied voltage, the scan rate, and the number of voltammogram scan cycles could be varied so that the background polymer deposition upon the conductive support does not hamper the detection of the polymer formed on the nanostructures.

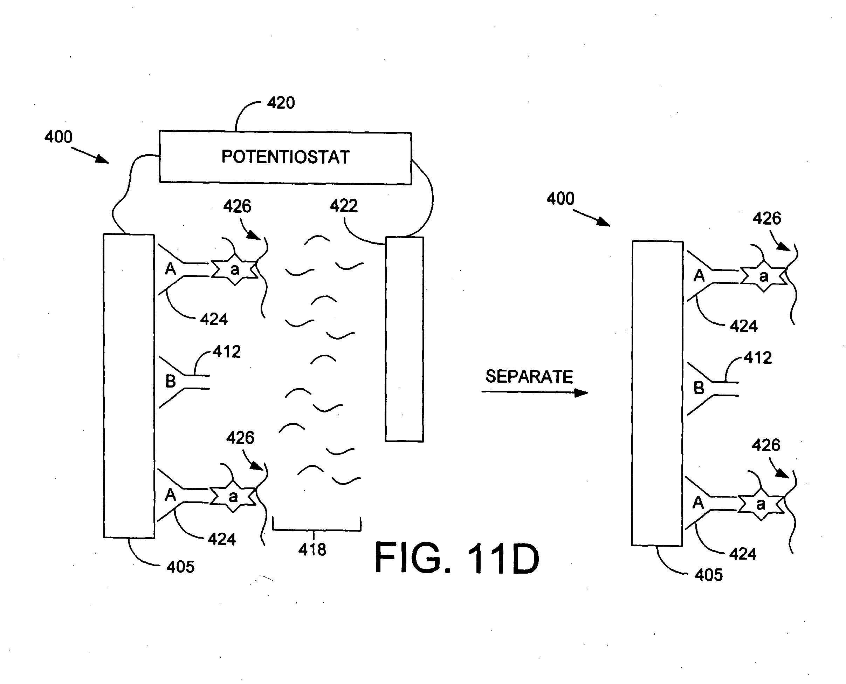

FIG. 11D is a schematic diagram illustrating the separation (*e.g.*, rinsing) of the monomer 418 and the remaining solution from the target polymer complexes 424 and non-target biomolecules 412 disposed on the substrate 405. As discussed above, additional preparation and/or treatment of the target polymer complexes 424 can be performed to facilitate the detection of the target polymer complexes 424.

The target polymer complexes 424 can be detected using one or more detection systems capable of detecting the polymers 426 on the target polymer complexes 424, the nanoparticles 414, and/or the target biomolecules 410. The detection system can include detection techniques discussed above in reference to FIGS. 4A through 4E.

The concentrations of the monomer 418 used and the settings of the potentiostat 420 depend upon, at least, the chemical formulation of the monomer 418, the concentration of the monomer 418, the pH of the solution, the concentration of acid to achieve the appropriate pH, the temperature of the solution, the type of nanoparticle 414, the conductive support 405, and the concentration of the target biomolecule. Therefore, the conditions under which the electrochemical amplification of the target biomolecular image is performed can vary depending upon the design of the biosensor 400.

## Claims

1. A method of detecting biomolecules, comprising:
providing a first biomolecule (110);
providing a first nanoparticle (114);
forming a target complex (116) that includes the first nanoparticle (114) and the first biomolecule (110);
forming a biomolecule image that includes the target complex (116); and
amplifying the biomolecule image by catalytically forming a polymer (124) on the first nanoparticle (114) of the target complex (116).

2. **The method of claim 1, wherein amplifying includes:**
providing a solution of monomers (118);
causing the target complex (116) to contact a solution of monomers (118);
providing an oxidant (120);
catalyzing the polymerization of the monomers (118) on the target complex (116) by adding an oxidant (120) to the solution;
forming the polymer (124) on the target complex (116); and
detecting the polymer (124), which corresponds to the first biomolecule (110).

3. The method of claim 1, further comprising:
providing a substrate (205), wherein the target complex (216) is disposed on the substrate (205);
providing a solution of monomers (218);
causing the substrate (205) having the target complex (216) disposed on the substrate (205) to contact a solution of monomers (218);
providing an oxidant (220);
catalyzing the polymerization of the monomers (218) on the target complex (216) by adding an oxidant (220) to the solution;
forming the polymer (224) on the target complex (216); and
detecting the polymer (224), which corresponds to the first biomolecule (210).

4. The method of claim 1, further comprising:
providing a solution of monomers (418);
contacting the conductive support (405) with the solution of monomers (418);
catalyzing the polymerization of the monomers (418) on the target complex (416) by applying a voltage to the conductive support (405);
forming the polymer (426) on the target complex (416); and
detecting the polymer (426), which corresponds to the first biomolecule (410).

5. The method of claim 4, wherein the monomer (418) is chosen from pyrroyl complexes, thiophene complexes, aniline complexes, fluorene complexes, carbazole complexes, and catechol (1,2-benzenediol) complexes.

6. A biosensor system for amplifying an image of a first biomolecule, comprising:
a first biomolecule (110);
a nanoparticle (116) disposed on the first biomolecule (110);
a polymer (124) disposed on the nanoparticle (116), wherein the polymer (124) is catalytically generated on the nanoparticle (116) from a plurality of monomers (118); and
a detector to detect the polymer (124).

7. The system of claim 6, wherein the monomer (118) is chosen from pyrroyl complexes, thiophene complexes, aniline complexes, fluorene complexes, carbazole complexes, and catechol (1,2-benzenediol) complexes.

8. The system of claim 6 or 7, further comprising an oxidant (120) to catalytically generate the monomer (118), wherein the oxidant (120) is chosen from transition metal salts and persulfate compounds.

9. The system of any of claims 6 to 8, wherein the first nanoparticle (114) includes a conjugated polymer nanoparticles in the size range from about 1 nanometer to about 1000 nanometers.

10. The system of any of claims 6 to 9, further comprising a potentiostat (420) capable of catalytically generating the polymer (426) by applying a voltage from about 100 to about 1500 millivolts (versus silver/silver chloride reference electrode) and wherein the first biomolecule (410) is attached to a conductive support (405).
